# EUROPEAN PATENT APPLICATION

(11) **EP 4 821 967 A1**
(43) Date of publication of application: **14.10.2026**
(21) Application number: 24900521.6
(22) Date of filing: 27.11.2024
(51) Int. Cl.: C08G 65/04, C07D 407/14, C08G 59/32, C08G 59/68, C09J 11/06, C09J 163/00

(54) **CURABLE COMPOSITION, CURED PRODUCT, ADHESIVE, AND SEALANT**

(30) Priority: 08.12.2023 JP 2023207547; 02.08.2024 JP 2024126468
(71) Applicant: SHIKOKU CHEMICALS CORPORATION, Marugame-shi, Kagawa 763-8504 (JP)
(72) Inventor: SHIOIRI, Ryosuke, Ayauta-gun, Kagawa 769-0202 (JP); ANDO, Syunsuke, Ayauta-gun, Kagawa 769-0202 (JP); KASHIWABARA, Takashi, Ayauta-gun, Kagawa 769-0202 (JP); AOKI, Kazunori, Ayauta-gun, Kagawa 769-0202 (JP); KUMANO, Takeshi, Ayauta-gun, Kagawa 769-0202 (JP)
(74) Representative: Graf von Stosch Patentanwaltsgesellschaft mbH
(86) International application number: PCT/JP2024/042030
(87) International publication number: WO 2025/121227

(57) **Abstract**

Provided are a curable composition having curability, low viscosity, and mechanical strength at a high level, a cured product of the curable composition, an adhesive, and a sealant. The curable composition contains an epoxy-oxetane compound represented by Chemical Formula (I): wherein Rs are identical or different and each represent a hydrogen atom or an alkyl group having 1 to 3 carbon atoms.

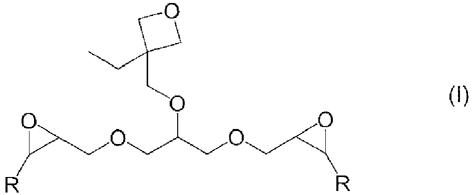

## Description

### Technical Field

The present invention relates to a curable composition, a cured product, an adhesive, and a sealant.

### Background Art

An epoxy-oxetane compound has recently attracted attention as a photocurable and heat-curable monomer. A curable composition containing an epoxy-oxetane compound as a component exhibits low shrinkage during curing, and a cured product (resin) of the curable composition is excellent in, for example, toughness, mechanical properties (mechanical strength), heat resistance, electrical properties, water resistance (moisture resistance), weather resistance, transparency, and adhesion (adhesiveness) to other materials.

Due to such excellent features, curable compositions containing an epoxy-oxetane compound are being promoted for use as raw materials for, for example, coating materials, paints, inks, adhesive materials (adhesives), pressuresensitive adhesive materials, films, pastes, optical materials, sealing materials (sealants), and resist materials.

Prior art related to the present invention is described below with reference to documents.

The invention disclosed in Patent Literature 1 relates to an active energy ray-curable ink composition containing a polymerizable compound having a molecular weight of 600 or less and containing three or more cationically polymerizable groups in a molecule.

Patent Literature 1 discloses, as polymerizable compounds, an epoxy compound represented by Chemical Formula (Ref-1) and an epoxy-oxetane compound represented by Chemical Formula (Ref-2).

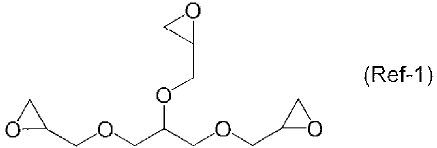

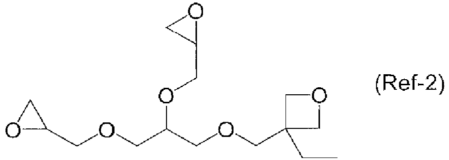

The invention disclosed in Patent Literature 2 relates to an adhesive composition for a thin film which adhesive composition contains a cyclic ether compound having an epoxy group and an oxetanyl group.

Patent Literature 2 discloses, as an example of the cyclic ether compound having an epoxy group and an oxetanyl group, an epoxy-oxetane compound represented by Chemical Formula (Ref-3).

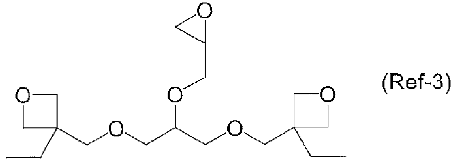

Curable compositions containing an epoxy-oxetane compound and used in various applications are required to (i) yield a cured product by curing as a characteristic thereof and (ii) have low viscosity from the viewpoint of workability during preparation of the curable composition and during use of the curable composition. Further, a cured product obtained by curing the curable composition is required to have, as characteristics thereof, high elasticity, excellent rigidity, high flexural strength, and excellent mechanical strength due to formation of a uniform three-dimensional network with few structural defects in the cured product. Curable compositions containing the epoxy-oxetane compounds represented by Chemical Formulas (Ref-1) to (Ref-3) and cured products of the curable compositions do not have those characteristics at a high level, and have a problem with curability, viscosity, and/or mechanical strength.

### Citation List

### [Patent Literature]

[Patent Literature 1]
   Japanese Patent Application Publication Tokukai No. 2010-111713
[Patent Literature 2]
   Japanese Patent Application Publication Tokukai No. 2019-189789

### Summary of Invention

### Technical Problem

An object of an aspect of the present invention is to provide a curable composition having curability, low viscosity, and mechanical strength at a high level, a cured product of the curable composition, an adhesive, and a sealant.

### Solution to Problem

An aspect of the present invention is a curable composition containing an epoxy-oxetane compound represented by Chemical Formula (I): 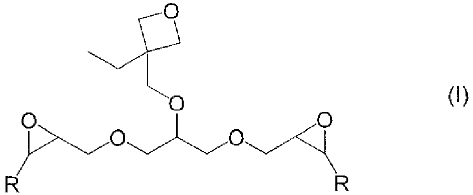 wherein Rs are identical or different and each represent a hydrogen atom or an alkyl group having 1 to 3 carbon atoms.

### Advantageous Effects of Invention

A curable composition containing an epoxy-oxetane compound represented by Chemical Formula (I) can be a curable composition having excellent curability, low viscosity, and excellent workability. Further, a cured product obtained by curing the curable composition can be a cured product having high elasticity, excellent rigidity, high flexural strength, and excellent mechanical strength due to formation of a uniform three-dimensional network with few structural defects in the cured product.

### Brief Description of Drawings

Fig. 1 is an IR spectrum chart of a pale yellow transparent liquid obtained in Synthesis Example 3.
Fig. 2 is an IR spectrum chart of a colorless transparent liquid obtained in Synthesis Example 4.
Fig. 3 is an IR spectrum chart of a pale yellow transparent liquid obtained in Synthesis Example 7.
Fig. 4 is an IR spectrum chart of a colorless transparent liquid obtained in Synthesis Example 10.
Fig. 5 is an IR spectrum chart of a pale yellow transparent liquid obtained in Synthesis Example 12.

### Description of Embodiments

A curable compound according to an aspect of the present invention contains an epoxy-oxetane compound represented by Chemical Formula (I). The epoxy-oxetane compound represented by Chemical Formula (I) has a structure in which two epoxy groups are symmetrically arranged with respect to one oxetane ring having an ethyl group, and the oxetane ring and each of the two epoxy groups are bonded via a connector having one ether bond. 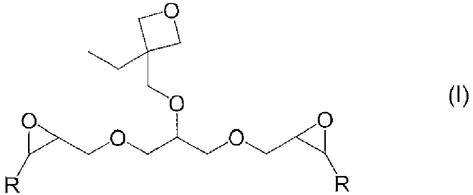 wherein Rs are identical or different and each represent a hydrogen atom or an alkyl group having 1 to 3 carbon atoms. The alkyl group may be linear or branched. Specific examples of the alkyl group include a methyl group, an ethyl group, an n-propyl group, and an isopropyl group. From the viewpoints of availability of raw materials and simplification of a synthesis process, Rs are preferably identical and are each preferably a hydrogen atom or a methyl group.

Examples of the epoxy-oxetane compound represented by Chemical Formula (I) include compounds represented by Chemical Formulas (I-1) to (I-6).

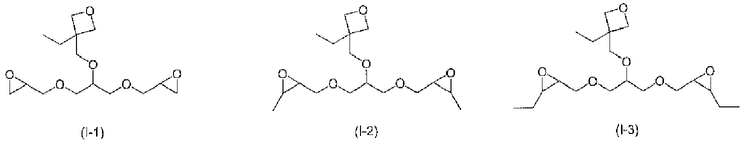

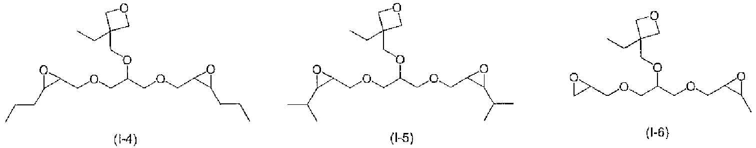

The epoxy-oxetane compound represented by Chemical Formula (I) is preferably any of epoxy-oxetane compounds represented by Chemical Formulas (I-1) to (I-6), and more preferably the epoxy-oxetane compound represented by Chemical Formula (I-1), from the viewpoints of availability of raw materials and simplification of a synthesis process. The epoxy-oxetane compound represented by Chemical Formula (I) may be used alone or in a mixture of two or more.

### <Method for synthesizing epoxy-oxetane compound represented by Chemical Formula (I)>

A bifunctional olefin compound represented by Chemical Formula (II) and having a hydroxy group is reacted with an oxetane compound represented by Chemical Formula (III) and having a leaving group to produce an olefin-oxetane compound represented by Chemical Formula (Ia), and then a double bond of the olefin-oxetane compound is epoxidized, so that the epoxy-oxetane compound represented by Chemical Formula (I) can be synthesized (see Reaction Scheme (A)).

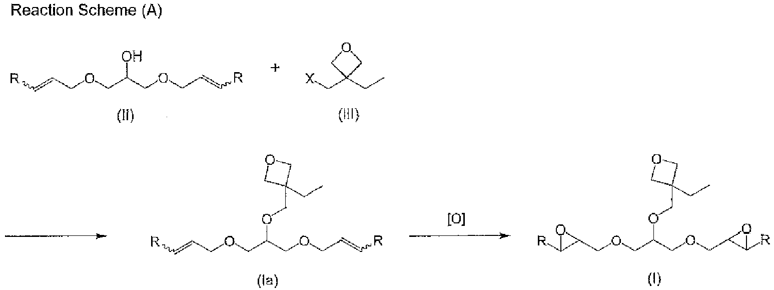

In Chemical Formula (III), X is a leaving group and represents a fluorine atom, a chlorine atom, a bromine atom, an iodine atom, a mesyloxy group (OMs), a tosyloxy group (OTs), or a trifluoromethanesulfonyloxy group (OTf). In Chemical Formulas (II) and (Ia), R has the same meaning as R in Chemical Formula (I), and a wavy line represents a trans isomer, a cis isomer, or a mixture thereof.

Examples of the oxetane compound represented by Chemical Formula (III) and having a leaving group include 3-ethyl-3-methanesulfonyloxymethyloxetane and 3-bromomethyl-3-ethyloxetane.

Examples of the bifunctional olefin compound represented by Chemical Formula (II) and having a hydroxy group include compounds represented by Chemical Formulas (II-1) to (II-15). The bifunctional olefin compound may be used alone or in combination of two or more.

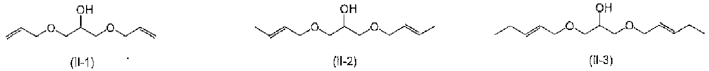

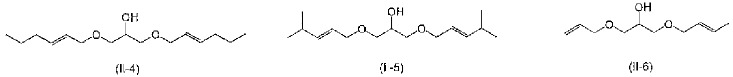

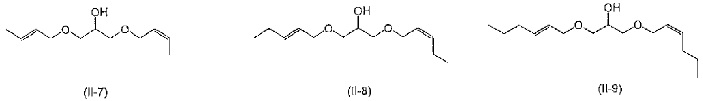

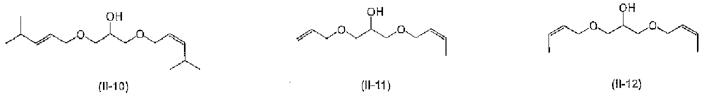

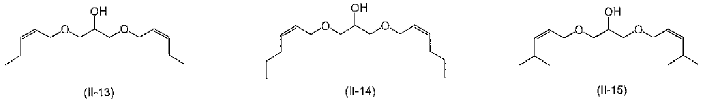

The bifunctional olefin compound represented by Chemical Formula (II) and having a hydroxy group can be synthesized by, for example, the method disclosed in Chinese Patent Application Publication No. 102267878. A biomass-derived raw material can also be used for synthesis of the bifunctional olefin compound, and the bifunctional olefin compound synthesized from a biomass-derived raw material is preferably used from the viewpoint of reducing environmental load. Specifically, a biomass-derived bifunctional olefin compound can be synthesized by using, as raw materials, biomass-derived 2-chloromethyloxirane (epichlorohydrin) and a biomass-derived olefin compound having a terminal hydroxy group.

The oxetane compound represented by Chemical Formula (III) and having a leaving group can be synthesized by, for example, the method disclosed in Japanese Patent Application Publication Tokukai No. 2007-332294. A biomass-derived raw material can also be used for synthesis of the oxetane compound, and the oxetane compound synthesized from a biomass-derived raw material is preferably used from the viewpoint of reducing environmental load. A biomass-derived epoxy-oxetane compound represented by Chemical Formula (I) can be synthesized by using, as raw materials, a biomass-derived bifunctional olefin compound represented by Chemical Formula (II) and having a hydroxy group and/or a biomass-derived oxetane compound represented by Chemical Formula (III) and having a leaving group.

The usage amount (charge amount) of the bifunctional olefin compound represented by Chemical Formula (II) and having a hydroxy group is preferably an appropriate proportion in a range of 0.8 molar equivalents to 2 molar equivalents with respect to the usage amount (charge amount) of the oxetane compound represented by Chemical Formula (III) and having a leaving group.

In a case where a bifunctional olefin compound represented by Chemical Formula (II-1) and a bifunctional olefin compound represented by Chemical Formula (II-2) are each used alone as the bifunctional olefin compound for a reaction, the epoxy-oxetane compound represented by Chemical Formula (I-1) and an epoxy-oxetane compound represented by Chemical Formula (I-2) can be respectively obtained.

The olefin-oxetane compound represented by Chemical Formula (Ia) can be synthesized in the presence of a base (i), and a catalyst (ii) for promoting a reaction may be used. Further, a reaction solvent (iii) may be used as long as the reaction is not inhibited.

In a reaction for epoxidizing a double bond of the olefin-oxetane compound represented by Chemical Formula (Ia), a common epoxidation (oxidation) method can be adopted, and examples thereof include a method using a peracid, a method using hydrogen peroxide with sodium tungstate as a catalyst, and a method using hydrogen peroxide together with a base in an acetonitrile-alcohol solvent.

Examples of the base (i) include hydrides, hydroxides, carbonates, hydrogen carbonates, alkoxides, and organic amine compounds of alkali metals and alkaline earth metals.

Examples of such bases include sodium hydride, potassium hydride, sodium hydroxide, potassium hydroxide, sodium carbonate, potassium carbonate, sodium alkoxide, potassium alkoxide (e.g., potassium t-butoxide), and triethylamine.

The usage amount (charge amount) of the base is ordinarily preferably an appropriate proportion in a range of 1.1 molar equivalents to 20 molar equivalents with respect to the usage amount (charge amount) of the oxetane compound having a leaving group.

Examples of the catalyst (ii) include quaternary ammonium salts and quaternary phosphonium salts.

Examples of the quaternary ammonium salts include salts such as halides (fluorides, chlorides, bromides, and iodides) of tetrabutylammonium, tetramethylammonium, tetraethylammonium, tetrapropylammonium, tetrahexylammonium, tetraoctylammonium, tetradecylammonium, hexadecyltriethylammonium, dodecyltrimethylammonium, trioctylmethylammonium, octyltriethylammonium, benzyltrimethylammonium, benzyltriethylammonium, benzyltributylammonium, benzyldimethyloctadecylammonium, and phenyltrimethylammonium.

Examples of the quaternary phosphonium salts include salts such as halides (fluorides, chlorides, bromides, and iodides) of tetrabutylphosphonium, tetramethylphosphonium, tetraethylphosphonium, tetrapropylphosphonium, tetrahexylphosphonium, tetradecylphosphonium, tetraoctylphosphonium, triethyloctadecylphosphonium, trioctylethylphosphonium, hexadecyltriethylphosphonium, tetraphenylphosphonium, and methyltriphenylphosphonium.

These substances may be used in combination as the catalyst (ii).

The usage amount (charge amount) of the catalyst (ii) is preferably an appropriate proportion in a range of 0.0001 molar equivalents to 1.0 molar equivalents with respect to the usage amount (charge amount) of the oxetane compound having a leaving group.

The reaction solvent (iii) is not particularly limited as long as the reaction is not inhibited. Examples of the reaction solvent (iii) include solvents such as water, tetrahydrofuran, diethyl ether, dioxane, 4-methyltetrahydropyran, dimethoxyethane, ethyl acetate, propyl acetate, butyl acetate, propylene glycol monomethyl ether acetate (PGMEA), acetonitrile, benzene, toluene, xylene, dichloromethane, chloroform, carbon tetrachloride, N,N-dimethylformamide (DMF), dimethylacetamide (DMA), dimethyl sulfoxide (DMSO), and hexamethylphosphoric triamide (HMPA), and one or a combination of two or more selected from these can be used in an appropriate amount.

The reaction temperature for synthesizing the olefin-oxetane compound represented by Chemical Formula (Ia) is preferably set in a range of 0°C to 150°C, and more preferably set in a range of 20°C to 120°C. The reaction time is set, as appropriate, in accordance with the set reaction temperature, and is preferably set in a range of 1 hour to 48 hours.

After completion of this reaction, an olefin-oxetane compound, which is a precursor of a target substance, can be separated and taken out from the obtained reaction liquid by, for example, a means such as a solvent extraction method.

Further, if necessary, the olefin-oxetane compound can be purified by using a means such as washing with water or the like, activated carbon treatment, or silica gel chromatography.

In the aforementioned reaction for epoxidizing the olefin-oxetane compound with use of a peracid, a peracid such as Oxone reagent, peracetic acid, or *m*-chloroperoxybenzoic acid (3-chloroperbenzoic acid) can be used. The usage amount (charge amount) of the peracid is preferably an appropriate proportion in a range of 1.0 molar equivalent to 5.0 molar equivalents with respect to a double bond of the olefin-oxetane compound.

In such an epoxidation reaction, the reaction solvent is not particularly limited as long as the reaction is not inhibited. Examples of the reaction solvent include: water; alcohols such as methanol, ethanol, and 2-propanol; aliphatic hydrocarbons such as hexane and heptane; ketones such as acetone and 2-butanone; esters such as ethyl acetate and butyl acetate; aromatic hydrocarbons such as benzene, toluene, and xylene; halogenated hydrocarbons such as methylene chloride (dichloromethane), chloroform, carbon tetrachloride, chlorotrifluoromethane, dichloroethane, chlorobenzene, and dichlorobenzene; ethers such as diethyl ether, diisopropyl ether, tetrahydrofuran, dioxane, dimethoxyethane, and diethylene glycol dimethyl ether; amides such as formamide, N,N-dimethylformamide (DMF), dimethylacetamide (DMA), N-methyl-2-pyrrolidone, N-methylpyrrolidinone, and hexamethylphosphorotriamide; and sulfoxides such as dimethyl sulfoxide (DMSO). These reaction solvents are used in an appropriate amount alone or in combination of two or more.

The reaction temperature in the epoxidation reaction is ordinarily in a range of -10°C to 150°C, and is preferably set in a range of 0°C to 100°C. The reaction time is set, as appropriate, in accordance with the reaction temperature, and is set ordinarily in a range of 1 hour to 48 hours, and preferably in a range of 1 hour to 24 hours.

After completion of this reaction, the epoxy-oxetane compound represented by Chemical Formula (I), which epoxy-oxetane compound is the target substance, can be separated and taken out from the obtained reaction liquid by, for example, a means such as a solvent extraction method.

Further, if necessary, the epoxy-oxetane compound can be purified by using a means such as washing with water or the like, activated carbon treatment, or silica gel chromatography.

In a reaction for epoxidizing the olefin-oxetane compound with use of hydrogen peroxide by using the sodium tungstate as a catalyst, the hydrogen peroxide is used at a proportion of 1.0 molar equivalent to 5.0 molar equivalents with respect to a double bond of the olefin-oxetane compound. The usage amount (charge amount) of the sodium tungstate is preferably an appropriate proportion in a range of 0.001 molar equivalents to 0.5 molar equivalents with respect to a double bond of the olefin-oxetane compound.

In such an epoxidation reaction, a reaction solvent is not particularly limited as long as the reaction is not inhibited. For example, reaction solvents similar to those in the case of the aforementioned epoxidation using a peracid can be used.

The reaction temperature in the epoxidation reaction is ordinarily in a range of -10°C to 150°C, and is preferably set in a range of 0°C to 100°C, as in the case of the aforementioned epoxidation using a peracid. The reaction time is set, as appropriate, in accordance with the reaction temperature, and is set ordinarily in a range of 1 hour to 48 hours, and preferably in a range of 1 hour to 24 hours.

After completion of the reaction, the epoxy-oxetane compound represented by Chemical Formula (I) can be separated and taken out from the obtained reaction liquid by, for example, a means such as a solvent extraction method, as in the case of the aforementioned epoxidation using a peracid. Further, if necessary, the epoxy-oxetane compound represented by Chemical Formula (I) may be purified.

In the aforementioned reaction for epoxidizing the olefin-oxetane compound with use of hydrogen peroxide together with a base in an acetonitrile-alcohol solvent, the usage amount (charge amount) of the hydrogen peroxide is preferably an appropriate proportion in a range of 1.0 molar equivalent to 5.0 molar equivalents with respect to a double bond of the olefin-oxetane compound.

The usage amount (charge amount) of acetonitrile is preferably an appropriate proportion in a range of 0.5 molar equivalents to 5.0 molar equivalents with respect to the olefin-oxetane compound.

The usage amount (charge amount) of alcohol is preferably an appropriate proportion in a range of 10% by weight to 80% by weight in a state before addition of the hydrogen peroxide. Further, it is preferable to use a base to set pH in a range of 7 to 13.

The alcohol used for such epoxidation is preferably a saturated alcohol having 1 to 4 carbon atoms, and examples thereof include methanol, ethanol, n-propanol, 2-propanol, n-butanol, sec-butanol (2-butanol), and isobutanol (2-methyl-1-propanol). These alcohols are used in an appropriate amount alone or in combination of two or more.

Examples of the base used for the epoxidation include hydroxides, carbonates, hydrogen carbonates, and organic amine compounds of alkali metals and alkaline earth metals, and sodium hydroxide, potassium hydroxide, potassium carbonate, and potassium hydrogen carbonate are preferably used in an appropriate amount alone or in combination of two or more.

The reaction temperature of the epoxidation is set ordinarily in a range of -10°C to 150°C, and preferably in a range of 0°C to 100°C, as in the case of the aforementioned epoxidation using a peracid. The reaction time is set, as appropriate, in accordance with the reaction temperature, and is set ordinarily in a range of 1 hour to 48 hours, and preferably in a range of 1 hour to 24 hours.

After completion of the reaction, the epoxy-oxetane compound represented by Chemical Formula (I) can be separated and taken out from the obtained reaction liquid by, for example, a means such as a solvent extraction method, as in the case of the aforementioned epoxidation using a peracid. Further, if necessary, the epoxy-oxetane compound represented by Chemical Formula (I) may be purified.

### <Curable composition according to an aspect of the present invention>

The epoxy-oxetane compound represented by Chemical Formula (I) exhibits excellent curability.

That is, by curing a curable composition containing the epoxy-oxetane compound represented by Chemical Formula (I) (hereinafter sometimes referred to as a "first curable compound"), it is possible to obtain a cured product (resin) that is expected to exhibit excellent characteristics.

In the curable composition according to an aspect of the present invention, it is also possible to use the first curable compound in combination with another curable compound different from the first curable compound (hereinafter sometimes referred to as a "second curable compound").

By allowing the second curable compound to coexist separately from the first curable compound during curing (polymerization) of the curable composition according to an aspect of the present invention, it is possible to obtain a cured product in which the first curable compound and the second curable compound are copolymerized.

Note that the second curable compound includes both a polymerizable monomer and a polymerizable oligomer (semicured product) having a structure in which the polymerizable monomer is polymerized.

Examples of the polymerizable monomer include known epoxy compounds (note: sometimes referred to as epoxy resins), oxetane compounds, epoxy-oxetane compounds (having an oxirane ring and an oxetane ring in a molecule), and acrylic compounds (note: sometimes referred to as acrylic resins).

As the epoxy compound, any epoxy compound having an oxirane ring (epoxy group/glycidyl group) in a molecule can be used without particular limitation. Examples of the epoxy compound include: polyglycidyl ethers obtained by reacting a polyhydric phenol such as bisphenol A, bisphenol F, bisphenol AD, catechol, or resorcinol, or a polyhydric alcohol such as glycerin or polyethylene glycol with epichlorohydrin; glycidyl ether esters obtained by reacting a hydroxycarboxylic acid such as p-hydroxybenzoic acid or β-hydroxynaphthoic acid with epichlorohydrin; polyglycidyl esters obtained by reacting a polycarboxylic acid such as phthalic acid or terephthalic acid with epichlorohydrin; glycidyl glycoluril compounds having two or more epoxy groups in a molecule, such as 1,3,4,6-tetraglycidyl glycoluril; alicyclic epoxy compounds such as 3',4'-epoxycyclohexylmethyl 3,4-epoxycyclohexanecarboxylate; nitrogen-containing cyclic epoxy compounds such as triglycidyl isocyanurate and hydantoin-type epoxy compounds; epoxidized phenol novolac resins; epoxidized cresol novolac resins; epoxidized polyolefins; cycloaliphatic epoxy resins; urethane-modified epoxy resins; and epoxy-modified organopolysiloxane compounds obtained by a hydrosilylation addition reaction of an organic compound having a carbon-carbon double bond and a glycidyl group with a silicon compound having an SiH group (e.g., epoxy-modified organopolysiloxane compounds disclosed in Japanese Patent Application Publication Tokukai No. 2004-99751 and Japanese Patent Application Publication Tokukai No. 2006-282988), and these may be used in combination.

As the oxetane compound, any oxetane compound having an oxetane ring (oxetanyl group/oxetane group) in a molecule can be used without particular limitation. Examples of the oxetane compound include 3-ethyl-3-hydroxymethyloxetane, 3-(meth)allyloxymethyl-3-ethyloxetane, (3-ethyl-3-oxetanylmethoxy)methylbenzene, 4-fluoro-[1-(3-ethyl-3-oxetanylmethoxy)methyl]benzene, 4-methoxy-[1 (3-ethyl-3-oxetanylmethoxy)methyl]benzene, [1-(3-ethyl-3-oxetanylmethoxy)ethyl]phenyl ether, isobutoxymethyl (3-ethyl-3-oxetanylmethyl) ether, isobornyloxyethyl (3-ethyl-3-oxetanylmethyl) ether, isobornyl (3-ethyl-3-oxetanylmethyl) ether, 2-ethylhexyl (3-ethyl-3-oxetanylmethyl) ether, ethyldiethylene glycol (3-ethyl-3-oxetanylmethyl) ether, dicyclopentadiene (3-ethyl-3-oxetanylmethyl) ether, dicyclopentenyloxyethyl (3-ethyl-3-oxetanylmethyl) ether, dicyclopentenyl (3-ethyl-3-oxetanylmethyl) ether, tetrahydrofurfuryl (3-ethyl-3-oxetanylmethyl) ether, 2-hydroxyethyl (3-ethyl-3-oxetanylmethyl) ether, 2-hydroxypropyl (3-ethyl-3-oxetanylmethyl) ether, butoxyethyl (3-ethyl-3-oxetanylmethyl) ether, bornyl (3-ethyl-3-oxetanylmethyl) ether, 3,7-bis(3-oxetanyl)-5-oxa-nonane, 3,3'-[1,3-(2-methylenyl)propanediylbis(oxymethylene)]bis-(3-ethyloxetane), 1,4-bis[(3-ethyl-3-oxetanylmethoxy)methyl]benzene, 1,2-bis[(3-ethyl-3-oxetanylmethoxy)methyl]ethane, 1,3-bis[(3-ethyl-3-oxetanylmethoxy)methyl]propane, ethylene glycol bis(3-ethyl-3-oxetanylmethyl) ether, dicyclopentenyl bis(3-ethyl-3-oxetanylmethyl) ether, triethylene glycol bis(3-ethyl-3-oxetanylmethyl) ether, tetraethylene glycol bis(3-ethyl-3-oxetanylmethyl) ether, tricyclodecanediyldimethylene (3-ethyl-3-oxetanylmethyl) ether, trimethylolpropane tris(3-ethyl-3-oxetanylmethyl) ether, 1,4-bis(3-ethyl-3-oxetanylmethoxy)butane, 1,6-bis(3-ethyl-3-oxetanylmethoxy)hexane, pentaerythritol tris(3-ethyl-3-oxetanylmethyl) ether, pentaerythritol tetrakis(3-ethyl-3-oxetanylmethyl) ether, polyethylene glycol bis(3-ethyl-3-oxetanylmethyl) ether, dipentaerythritol hexakis(3-ethyl-3-oxetanylmethyl) ether, dipentaerythritol pentakis(3-ethyl-3-oxetanylmethyl) ether, dipentaerythritol tetrakis(3-ethyl-3-oxetanylmethyl) ether, caprolactone-modified dipentaerythritol hexakis(3-ethyl-3-oxetanylmethyl) ether, caprolactone-modified dipentaerythritol pentakis(3-ethyl-3-oxetanylmethyl) ether, ditrimethylolpropane tetrakis(3-ethyl-3-oxetanylmethyl) ether, EO-modified bisphenol A bis(3-ethyl-3-oxetanylmethyl) ether, PO-modified bisphenol A bis(3-ethyl-3-oxetanylmethyl) ether, EO-modified hydrogenated bisphenol A bis(3-ethyl-3-oxetanylmethyl) ether, PO-modified hydrogenated bisphenol A bis(3-ethyl-3-oxetanylmethyl) ether, and EO-modified bisphenol F (3-ethyl-3-oxetanylmethyl) ether.

As the epoxy-oxetane compound, any epoxy-oxetane compound having an oxirane ring (epoxy group/glycidyl group) and an oxetane ring (oxetanyl group/oxetane group) in a molecule can be used without particular limitation. Examples of the epoxy-oxetane compound include epoxy-oxetane compounds disclosed in Patent Literatures 1 and 2. The epoxy-oxetane compounds disclosed in these documents are incorporated in the disclosure of the present specification by reference.

Examples of the acrylic compounds include allyl (meth)acrylate, vinyl (meth)acrylate, dicyclopentenyl (meth)acrylate, dicyclopentenyloxyethyl (meth)acrylate, (meth)acrylic acid-modified allyl glycidyl ether (manufactured by Nagase ChemteX Corporation, "Denacol Acrylate DA111 (trade name)"), urethane (meth)acrylates, epoxy (meth)acrylates, trimethylolpropane tri(meth)acrylate, pentaerythritol tetra(meth)acrylate, ditrimethylolpropane tetra(meth)acrylate, dipentaerythritol hexa(meth)acrylate, butanediol di(meth)acrylate, nonanediol di(meth)acrylate, polypropylene glycol-based (meth)acrylate, bisphenol A di(meth)acrylate, tris(2-(meth)acryloyloxyethyl) isocyanurate, and (meth)acrylate group-containing polyorganosiloxane.

In the curable composition according to an aspect of the present invention, the foregoing polymerizable monomer and polymerizable oligomer may be used in combination as the second curable compound. As the polymerizable monomer, the polymerizable monomers listed earlier may be used in combination (different types of polymerizable monomers may be used in combination), and also for the polymerizable oligomer, different types of polymerizable oligomers may be used in combination.

Regarding the ratio of the content of the first curable compound to the content of the second curable compound in the curable composition according to an aspect of the present invention, the content of the second curable compound is preferably an appropriate proportion in a range of 0 times to 1,000 times (weight ratio), and more preferably an appropriate proportion in a range of 0.01 times to 100 times (weight ratio), with respect to the content of the first curable compound.

Examples of a method for curing (polymerizing) the curable composition according to an aspect of the present invention include methods of photo-curing and heat-curing the curable composition.

Examples of the method of photo-curing the curable composition include a method of irradiating the curable composition with active energy rays and a method of using a photopolymerization initiator in combination. The active energy rays include, for example, light, radiation, electromagnetic waves, and electron beams, and typically represent light, particularly ultraviolet rays.

As the photopolymerization initiator, a photocationic polymerization initiator or a photoanionic polymerization initiator can be adopted. If necessary, a photoradical polymerization initiator can be used in combination with the photocationic polymerization initiator or the photoanionic polymerization initiator. The photocationic polymerization initiator or the photoanionic polymerization initiator and/or the photoradical polymerization initiator may be contained in the curable composition. Note that a heat curing means may be used in combination with photocuring in order to improve production efficiency and characteristics of a cured product.

As the photocationic polymerization initiator, any commonly used photocationic polymerization initiator can be used without particular limitation. Examples of the photocationic polymerization initiator include onium salts and organometallic complexes.

Examples of the onium salts include diazonium salts, sulfonium salts, and iodonium salts. Examples of the organometallic complexes include iron-allene complexes, titanocene complexes, and arylsilanol-aluminum complexes.

Examples of industrial chemicals commercially available as photocationic polymerization initiators include "Optomer SP-150 (trade name)" manufactured by ADEKA Corporation, "Optomer SP-170 (trade name)" manufactured by ADEKA Corporation, "CPI-100P (trade name)" manufactured by San-Apro Ltd., "UVE-1014 (trade name)" manufactured by General Electric Company, and "CD-1012 (trade name)" manufactured by Sartomer.

Examples of a counter anion of the photocationic polymerization initiator include SbF₆⁻, AsF₆⁻, B(C₆F₅)₄⁻, and PF₆⁻.

As the photoanionic polymerization initiator, any commonly used photoanionic polymerization initiator can be used without particular limitation. Examples of the photoanionic polymerization initiator include onium salts and carbamates.

Examples of the onium salts include 1,2-diisopropyl-3-(bis(dimethylamino)methylene)guanidinium 2-(3-benzoylphenyl)propionate and 1,2-dicyclohexyl-4,4,5,5-tetramethylbiguanidinium n-butyltriphenylborate.

Examples of the carbamates include 2-nitrophenylmethylpiperidine-1-carboxylate, 1-(anthraquinon-2-yl)ethylimidazolecarboxylate, 1-(3-(2-hydroxyphenyl)-2-propenoyl)piperidine, and 9-anthranylmethyldiethylcarbamate.

The content of the photocationic polymerization initiator or the photoanionic polymerization initiator in the curable composition according to an aspect of the present invention is preferably 0.001% by weight to 20% by weight, and more preferably 0.01% by weight to 10% by weight.

Examples of the photoradical polymerization initiator include ketal compounds having 16 to 17 carbon atoms (e.g., acetophenone dimethyl ketal, benzyl dimethyl ketal, etc.), acetophenone compounds having 8 to 18 carbon atoms (e.g., acetophenone, 2,2-dimethoxy-2-phenylacetophenone, 2,2-diethoxy-2-phenylacetophenone, 1,1-dichloroacetophenone, 2-hydroxy-2-methyl-phenylpropan-1-one, diethoxyacetophenone, 1-hydroxycyclohexyl phenyl ketone, 2-methyl-1-[4-(methylthio)phenyl]-2-morpholinopropan-1-one, etc.), benzophenone compounds having 13 to 21 carbon atoms (e.g., benzophenone, 4-benzoyl-4'-methyldiphenyl sulfide, 4,4'-bismethylaminobenzophenone, etc.), benzoin compounds having 14 to 18 carbon atoms (e.g., benzoin, benzoin methyl ether, benzoin ethyl ether, benzoin propyl ether, benzoin isobutyl ether, etc.), anthraquinone compounds having 14 to 19 carbon atoms (e.g., 2-ethylanthraquinone, 2-tert-butylanthraquinone, 2-chloroanthraquinone, 2-amylanthraquinone, etc.), thioxanthone compounds having 13 to 17 carbon atoms (e.g., 2,4-diethylthioxanthone, 2-isopropylthioxanthone, 2-chlorothioxanthone, etc.), and acylphosphine oxide compounds having 22 to 28 carbon atoms (e.g., 2,4,6-trimethylbenzoyl-diphenyl-phosphine oxide, bis-(2,6-dimethoxybenzoyl)-2,4,4-trimethylpentylphosphine oxide, bis(2,4,6-trimethylbenzoyl)-phenylphosphine oxide, etc.).

The content of the photoradical polymerization initiator in the curable composition according to an aspect of the present invention is preferably 0.001% by weight to 20% by weight, and more preferably 0.01% by weight to 10% by weight.

For example, a sensitizer such as pyrene, perylene, acridine orange, thioxanthone, 2-chlorothioxanthone, or benzoflavin can be used to photo-cure the curable composition according to an aspect of the present invention.

In contrast, a thermal polymerization initiator can be used to heat-cure the curable composition according to an aspect of the present invention. As the thermal polymerization initiator, a thermal cationic polymerization initiator, a thermal radical polymerization initiator, or a thermal anionic polymerization initiator can be adopted, and such a thermal polymerization initiator may be contained in the curable composition.

As the thermal cationic polymerization initiator, any commonly used thermal cationic polymerization initiator can be used without particular limitation. Examples of the thermal cationic polymerization initiator include various onium salts such as quaternary ammonium salts, phosphonium salts, and sulfonium salts, and organometallic complexes.

Examples of onium salts commercially available as industrial chemicals include "Adeka Opton CP-66 (trade name)" manufactured by ADEKA Corporation, "Adeka Opton CP-77 (trade name)" manufactured by ADEKA Corporation, "San-Aid SI-60L (trade name)" manufactured by Sanshin Chemical Industry Co., Ltd., "San-Aid SI-80L (trade name)" manufactured by Sanshin Chemical Industry Co., Ltd., "San-Aid SI-100L (trade name)" manufactured by Sanshin Chemical Industry Co., Ltd., and "CI Series (trade name)" manufactured by Nippon Soda Co., Ltd.

Examples of the organometallic complexes include alkoxysilane-aluminum complexes.

As the thermal radical polymerization initiator, any commonly used thermal radical polymerization initiator can be used without particular limitation. Examples of the thermal radical polymerization initiator include: peroxides such as diisopropyl peroxydicarbonate, benzoyl peroxide, t-butyl peroxyisobutyrate, t-hexyl peroxyisopropyl monocarbonate, t-hexyl peroxy 2-ethylhexanoate, 1,1,3,3-tetramethylbutyl peroxy 2-ethylhexanoate, t-butyl peroxypivalate, t-hexyl peroxypivalate, t-butyl peroxyneodecanoate, t-hexyl peroxyneodecanoate, 1,1,3,3-tetramethylbutyl peroxyneodecanoate, 1,1-bis(t-hexylperoxy)cyclohexane, benzoyl peroxide, 3,5,5-trimethylhexanoyl peroxide, and lauroyl peroxide; and azo compounds such as azobisisobutyronitrile, 2,2'-azobis(2-methylbutyronitrile), and dimethyl 2,2'-azobis(2-methylpropionate), and these may be used in combination.

As the thermal anionic polymerization initiator, any commonly used thermal anionic polymerization initiator can be used without particular limitation. Examples of the thermal anionic polymerization initiator include amines and imidazoles, and these may be used in combination.

Regarding conditions for heat curing, the heating temperature/heating time can be set, as appropriate, and is preferably set in a range of 60°C to 200°C/30 minutes to 240 minutes, and more preferably set in a range of 70°C to 180°C/30 minutes to 120 minutes.

The content of the thermal polymerization initiator in the curable composition according to an aspect of the present invention is preferably 0.001% by weight to 20% by weight, and more preferably 0.01% by weight to 10% by weight.

In the present invention, a cationic polymerization initiator refers to the aforementioned photocationic polymerization initiator and/or the aforementioned thermal cationic polymerization initiator, the anionic polymerization initiator refers to the aforementioned photoanionic polymerization initiator and/or the aforementioned thermal anionic polymerization initiator, and the radical polymerization initiator refers to the aforementioned photoradical polymerization initiator and/or the aforementioned thermal radical polymerization initiator.

Another method for curing (polymerizing) the curable composition according to an aspect of the present invention includes a method of causing the curable composition to contain a curing agent and photo-curing and heat-curing the curable composition. In a case where the curable composition is heat-cured, a thermal polymerization initiator can be used in combination with the curing agent.

Examples of the curing agent include acid anhydride compounds, thiol compounds, and amine compounds.

Examples of the acid anhydride compounds include methyltetrahydrophthalic anhydride, methylhexahydrophthalic anhydride, hexahydrophthalic anhydride, 5-norbornene-2,3-dicarboxylic anhydride, trimellitic anhydride, nadic anhydride, himic anhydride, methylnadic anhydride, methylbicyclo[2.2.1]heptane-2,3-dicarboxylic anhydride, bicyclo[2.2.1]heptane-2,3-dicarboxylic anhydride, and methylnorbornane-2,3-dicarboxylic acid anhydride.

Examples of the thiol compounds include: aliphatic thiol compounds such as ethanedithiol, propanedithiol, hexamethylenedithiol, decamethylenedithiol, tolylene-2,4-dithiol, 2,2-bis(mercaptomethyl)-1,3-propanedithiol, 2-(mercaptomethyl)-2-methyl-1,3-propanedithiol, and 2-ethyl-2-(mercaptomethyl)-1,3-propanedithiol; cyclic sulfide compounds such as a 1,4-dithiane ring-containing polythiol compound represented by Formula (XIV); mercaptoalkyl sulfide compounds such as 3-thiapentane-1,5-dithiol and 4-mercaptomethyl-3,6-dithia-1,8-octanedithiol; mercaptopropionic acid esters such as pentaerythritol tetrakis(3-mercaptopropionate); epoxy resin terminal mercapto compounds; mercaptoalkyl ether compounds such as 3,6-dioxa-1,8-octanedithiol, a mercaptoalkyl ether disulfide compound represented by Formula (XV), 2,2'-[[2,2-bis[(2-mercaptoethoxy)methyl]-1,3-propanediyl]bis(oxy)]bisethanethiol, 3,3'-[[2,2-bis[(3-mercaptopropoxy)methyl]-1,3-propanediyl]bis(oxy)]bis-1-propanethiol, 3-[2,2-bis[(3-mercaptopropoxy)methyl]butoxy]-1-propanethiol, 3-(3-mercaptopropoxy)-2,2-bis[(3-mercaptopropoxy)methyl]-1-propanol, and 2,2-bis[(3-mercaptopropoxy)methyl]-1-butanol; 1,3,4,6-tetrakis(2-mercaptoethyl)glycoluril; and 1,3,4,6-tetrakis(3-mercaptopropyl)glycoluril, and 1,3,4,6-tetrakis(2-mercaptoethyl)glycoluril and 1,3,4,6-tetrakis(3-mercaptopropyl)glycoluril are preferable.

These other thiol compounds may be used alone or in combination of two or more. 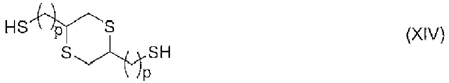 where p represents an integer of 1 to 5. 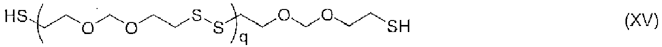 where q represents an integer of 1 to 20.

As conventionally known, an amine compound may be any amine compound having, in a molecule, at least one amino group selected from a primary amino group, a secondary amino group, and a tertiary amino group.

Examples of such an amine compound include: aliphatic amines such as diethylenetriamine, triethylenetetramine, isophoronediamine, xylylenediamine, diaminodiphenylmethane, 1,3,4,6-tetrakis(3-aminopropyl)glycoluril, n-propylamine, 2-hydroxyethylaminopropylamine, cyclohexylamine, 4,4'-diaminodicyclohexylmethane, and dimethylbenzylamine; aromatic amines such as 4,4'-diaminodiphenylmethane and o-methylaniline; and nitrogen-containing heterocyclic compounds such as 2-ethyl-4-methylimidazole, 2-methylimidazole, 2-ethyl-4-methylimidazoline, 2,4-dimethylimidazoline, piperidine, and piperazine.

The content of the curing agent in the curable composition according to an aspect of the present invention is preferably 0.1 parts by weight to 50 parts by weight, and more preferably 1 part by weight to 40 parts by weight, with respect to 100 parts by weight of the epoxy-oxetane compound.

A reaction product of the epoxy compound and the amine compound, or a reaction product of a compound having one or more isocyanate groups in a molecule and a compound having a primary amino group and/or a secondary amino group in a molecule is a solid that is poorly soluble in an epoxy resin at room temperature, but is solubilized (easily solubilized) by heating and functions as a curing accelerator, and thus is also referred to as a latent curing accelerator (hereinafter, such a reaction product is sometimes referred to as a "latent curing accelerator").

Examples of the epoxy compound used as a raw material for the latent curing accelerator composed of the reaction product of the epoxy compound and the amine compound include, in addition to the aforementioned epoxy compounds, glycidylamine compounds obtained by reacting 4,4'-diaminodiphenylmethane, m-aminophenol, or the like with epichlorohydrin; and monofunctional epoxy compounds such as butyl glycidyl ether, phenyl glycidyl ether, and glycidyl methacrylate.

Examples of the amine compound used as the raw material for the latent curing accelerator composed of the reaction product of the epoxy compound and the amine compound include the aforementioned amine compounds. Among these amine compounds, an amine compound having a tertiary amino group in a molecule is a raw material that gives a latent curing accelerator having excellent curing acceleration properties. Examples of such an amine compound include: amines such as dimethylaminopropylamine, diethylaminopropylamine, di-n-propylaminopropylamine, dibutylaminopropylamine, dimethylaminoethylamine, diethylaminoethylamine, and N-methylpiperazine; amines having a tertiary amino group in a molecule, such as imidazole compounds like 2-methylimidazole, 2-ethylimidazole, 2-ethyl-4-methylimidazole, 2-phenylimidazole, etc.; and alcohols, phenols, thiols, carboxylic acids, and hydrazides, each having a tertiary amino group in a molecule, such as 2-dimethylaminoethanol, 1-methyl-2-dimethylaminoethanol, 1-phenoxymethyl-2-dimethylaminoethanol, 2-diethylaminoethanol, 1-butoxymethyl-2-dimethylaminoethanol, 1-(2-hydroxy-3-phenoxypropyl)-2-methylimidazole, 1-(2-hydroxy-3-phenoxypropyl)-2-ethyl-4-methylimidazole, 1-(2-hydroxy-3-butoxypropyl)-2-methylimidazole, 1-(2-hydroxy-3-butoxypropyl)-2-ethyl-4-methylimidazole, 1-(2-hydroxy-3-phenoxypropyl)-2-phenylimidazoline, 1-(2-hydroxy-3-butoxypropyl)-2-methylimidazoline, 2-(dimethylaminomethyl)phenol, 2,4,6-tris(dimethylaminomethyl)phenol, N-β-hydroxyethylmorpholine, 2-dimethylaminoethanethiol, 2-mercaptopyridine, 2-mercaptobenzimidazole, 2-mercaptobenzothiazole, 4-mercaptopyridine, N,N-dimethylaminobenzoic acid, N,N-dimethylglycine, nicotinic acid, isonicotinic acid, picolinic acid, N,N-dimethylglycine hydrazide, N,N-dimethylpropionic acid hydrazide, nicotinic acid hydrazide, and isonicotinic acid hydrazide.

In order to further improve storage stability of the curable composition according to an aspect of the present invention, an active hydrogen compound having two or more active hydrogens in a molecule may be used as a third component in addition to the aforementioned epoxy compound and the aforementioned amine compound as the raw materials for the latent curing accelerator composed of the reaction product of the epoxy compound and the amine compound.

Examples of the active hydrogen compound include: polyhydric phenols such as bisphenol A, bisphenol F, bisphenol S, hydroquinone, catechol, resorcinol, pyrogallol, and phenol novolac resin; polyhydric alcohols such as trimethylolpropane; polycarboxylic acids such as adipic acid and phthalic acid; 1,2-dimercaptoethane; 2-mercaptoethanol; 1-mercapto-3-phenoxy-2-propanol; mercaptoacetic acid; anthranilic acid; and lactic acid.

Further, the latent curing accelerator composed of the reaction product of the epoxy compound and the amine compound may be surface-treated with an isocyanate compound or an acidic compound. Examples of the isocyanate compound include: monofunctional isocyanate compounds such as n-butyl isocyanate, isopropyl isocyanate, phenyl isocyanate, and benzyl isocyanate; and polyfunctional isocyanate compounds such as hexamethylene diisocyanate, toluylene diisocyanate, 1,5-naphthalene diisocyanate, diphenylmethane-4,4'-diisocyanate, isophorone diisocyanate, xylylene diisocyanate, paraphenylene diisocyanate, 1,3,6-hexamethylene triisocyanate, and bicycloheptane triisocyanate.

Instead of such a polyfunctional isocyanate compound, a terminal isocyanate group-containing compound obtained by reacting a polyfunctional isocyanate compound with an active hydrogen compound can also be used in the same manner as the polyfunctional isocyanate compound. Examples of such a terminal isocyanate group-containing compound include a terminal isocyanate group-containing addition reaction product obtained by reacting toluylene diisocyanate with trimethylolpropane, and a terminal isocyanate group-containing addition reaction product obtained by reacting toluylene diisocyanate with pentaerythritol.

The acidic compound used for surface treatment of the latent curing accelerator composed of the reaction product of the epoxy compound and the amine compound may be any of gas, liquid, and solid, and may be either inorganic acid or organic acid. Examples of such an acidic compound include carbon dioxide gas, sulfurous acid gas, sulfuric acid, hydrochloric acid, oxalic acid, phosphoric acid, acetic acid, formic acid, propionic acid, adipic acid, caproic acid, lactic acid, succinic acid, tartaric acid, sebacic acid, p-toluenesulfonic acid, salicylic acid, boric acid, tannic acid, alginic acid, polyacrylic acid, polymethacrylic acid, phenol, pyrogallol, phenol resin, and resorcinol resin.

The latent curing accelerator composed of the reaction product of the epoxy compound and the amine compound can be easily obtained by mixing the epoxy compound, the amine compound, and, if necessary, an active hydrogen compound, reacting the mixture thus obtained at a temperature from room temperature to 200°C, and then solidifying and pulverizing the mixture, or reacting the mixture in a solvent such as methyl ethyl ketone, dioxane, or tetrahydrofuran, removing the solvent, and then pulverizing a solid content.

Further, a commercially available latent curing accelerator composed of the reaction product of the epoxy compound and the amine compound can also be used. Examples of commercially available products of the latent curing accelerator include: "Amicure PN-23 (trade name)", "Amicure PN-H (trade name)", "Amicure PN-50 (trade name)", "Amicure PN-23J (trade name)", "Amicure PN-40J (trade name)", and "Amicure MY-24 (trade name)", each manufactured by Ajinomoto Fine-Techno Co., Inc.; "Novacure HX-3088 (trade name)", "Novacure HX-3721 (trade name)", "Novacure HX-3722 (trade name)", "Novacure HX-3742 (trade name)", "Novacure HX-3941HP (trade name)", and "Novacure HXA3922HP (trade name)", each manufactured by Asahi Kasei Corporation; and "Fujicure FXR-1030 (trade name)", "Fujicure FXR-1081 (trade name)", and "Fujicure FXR-1121 (trade name)", each manufactured by T&K TOKA Corporation.

The content of the latent curing accelerator composed of the reaction product of the epoxy compound and the amine compound in the curable composition according to an aspect of the present invention is preferably 0.1 parts by weight to 100 parts by weight, more preferably 1 part by weight to 90 parts by weight, and even more preferably 1 part by weight to 80 parts by weight, with respect to 100 parts by weight of the epoxy-oxetane compound.

Examples of an isocyanate compound having one or more isocyanate groups in a molecule and used as a raw material for the latent curing accelerator composed of the reaction product of the compound having one or more isocyanate groups in a molecule and the compound having a primary amino group and/or a secondary amino group in a molecule include n-butyl isocyanate, isopropyl isocyanate, 2-chloroethyl isocyanate, phenyl isocyanate, p-bromophenyl isocyanate, m-chlorophenyl isocyanate, o-chlorophenyl isocyanate, p-chlorophenyl isocyanate, 2,5-dichlorophenyl isocyanate, 3,4-dichlorophenyl isocyanate, 2,6-dimethylphenyl isocyanate, o-fluorophenyl isocyanate, p-fluorophenyl isocyanate, m-tolyl isocyanate, p-tolyl isocyanate, o-trifluoromethylphenyl isocyanate, m-trifluoromethylphenyl isocyanate, benzyl isocyanate, hexamethylene diisocyanate, 2,4-toluylene diisocyanate, 2,6-toluylene diisocyanate, 1,5-naphthalene diisocyanate, diphenylmethane-4,4'-diisocyanate, 2,2-dimethyldiphenylmethane-4,4'-diisocyanate, tolidine diisocyanate, isophorone diisocyanate, xylylene diisocyanate, 1,3-bis(isocyanatomethyl)cyclohexane, p-phenylene diisocyanate, 1,3,6-hexamethylene triisocyanate, bicycloheptane triisocyanate, tris-(3-isocyanato-4-methylphenyl)isocyanurate, and tris-(6-isocyanatohexyl)isocyanurate.

Examples of the compound having a primary amino group and/or a secondary amino group in a molecule and used as a raw material for the latent curing accelerator composed of the reaction product of the compound having one or more isocyanate groups in a molecule and the compound having a primary amino group and/or a secondary amino group in a molecule include: dimethylamine, diethylamine, di-n-propylamine, di-n-butylamine, di-n-hexylamine, di-n-octylamine, di-n-ethanolamine, dimethylaminopropylamine, diethylaminopropylamine, morpholine, piperidine, 2,6-dimethylpiperidine, 2,2,6,6-tetramethylpiperidine, piperazine, pyrrolidine, benzylamine, N-methylbenzylamine, cyclohexylamine, metaxylylenediamine, 1,3-bis(aminomethyl)cyclohexane, isophoronediamine, N-aminoethylpiperazine, 2-methylimidazole, 2-ethyl-4-methylimidazole, 2-undecylimidazole, 2-phenylimidazole, and 1,1-dimethylhydrazine.

The reaction product of the compound having one or more isocyanate groups in a molecule and the compound having a primary amino group and/or a secondary amino group in a molecule can be obtained by reacting, in an organic solvent such as dichloromethane, both the compound having one or more isocyanate groups in a molecule and the compound having a primary amino group and/or a secondary amino group in a molecule.

The content of the latent curing accelerator composed of the reaction product of the compound having one or more isocyanate groups in a molecule and the compound having a primary amino group and/or a secondary amino group in a molecule in the curable composition according to an aspect of the present invention is preferably 1 part by weight to 10 parts by weight with respect to 100 parts by weight of the epoxy-oxetane compound.

The curable composition according to an aspect of the present invention may further contain additives (modifiers) such as pigments (titanium white, cyanine blue, Watchung Red, red iron oxide, carbon black, aniline black, manganese blue, iron black, ultramarine blue, Hansa red, chrome yellow, chrome green, etc.), inorganic fillers (calcium carbonate, kaolin, clay, talc, mica, barium sulfate, lithopone, gypsum, zinc stearate, perlite, quartz, quartz glass, fused silica, silica powder such as spherical silica, spherical alumina, crushed alumina, oxides such as magnesium oxide, beryllium oxide, and titanium oxide, nitrides such as boron nitride, silicon nitride, and aluminum nitride, carbides such as silicon carbide, hydroxides such as aluminum hydroxide and magnesium hydroxide, metals and alloys such as copper, silver, iron, aluminum, nickel, and titanium, carbon-based materials such as diamond and carbon, etc.), thermoplastic resins and thermosetting resins (homopolymers such as various high-density, medium-density, and low-density polyethylenes, polypropylene, polybutene, and polypentene, ethylene-propylene copolymers, polyamide resins such as nylon-6 and nylon-6,6, vinyl chloride resins, nitrocellulose resins, vinylidene chloride resins, acrylamide resins, styrene resins, vinyl ester resins, polyester resins, phenol resins (phenol compounds), silicone resins, fluorine resins, various elastomer resins such as acrylic rubber and urethane rubber, graft copolymers such as methyl methacrylate-butadiene-styrene graft copolymers and acrylonitrile-butadiene-styrene graft copolymers, etc.), reinforcing agents (glass fiber, carbon fiber, etc.), anti-sagging agents (hydrogenated castor oil, fine particle anhydrous silicic acid, etc.), matting agents (fine powder silica, paraffin wax, etc.), abrasives (zinc stearate, etc.), internal mold release agents (fatty acids such as stearic acid, fatty acid metal salts of calcium stearate, fatty acid amides such as stearic acid amide, fatty acid esters, polyolefin wax, paraffin wax, etc.), surfactants, leveling agents, antifoaming agents, viscosity adjusting diluents (organic solvents), coupling agents, fragrances, and flame retardants, as long as the effects of the present invention are not impaired.

A method for preparing the curable composition according to an aspect of the present invention is not particularly limited, and the curable composition is prepared by weighing predetermined amounts of the foregoing components and stirring and mixing the components. For example, the curable composition according to an aspect of the present invention can be prepared by premixing the components and then using a roll kneader, a kneader, an extruder, or the like to mix or melt-knead the components. If necessary, an organic solvent (viscosity adjusting diluent) may be used.

The curable composition according to an aspect of the present invention is polymerized (cured) by irradiation with ultraviolet rays or heating to give a cured product.

Examples of a means for irradiation with ultraviolet rays include a method using a light source such as a chemical lamp, a low-pressure mercury lamp, a high-pressure mercury lamp, an ultra-high-pressure mercury lamp, a xenon lamp, or a metal halide lamp.

The irradiation intensity and irradiation time of ultraviolet rays are set, as appropriate, in consideration of a desired irradiation intensity or a desired irradiation time and also in consideration of the composition and shape (thickness) of the curable composition to be irradiated.

Examples of a heating means include methods such as hot air circulation, infrared heating, and high-frequency heating. As a curing device, a closed curing furnace, a tunnel furnace capable of continuous curing, or the like can be used.

The heating (curing) temperature and heating (curing) time may be set, as appropriate, in consideration of the composition and shape (thickness) of the curable composition to be irradiated, as in the case of ultraviolet irradiation.

The curable composition according to an aspect of the present invention has low viscosity, and thus can be used as a curable ink composition. Furthermore, the curable composition according to an aspect of the present invention has low viscosity and excellent dischargeability. Thus, the curable composition according to an aspect of the present invention is also suitable as an inkjet curable ink composition. The inkjet curable ink composition that is applied to a predetermined object by using an inkjet method and cured is used for manufacturing various components and members (described later). The inkjet method makes it possible to form a resin film at high speed and uniformly over a large area, and thus has advantages such as improvement in product accuracy of various components and members (described later) and reduction in manufacturing cost.

The curable ink composition and the inkjet curable ink composition preferably have a 5% weight loss temperature (°C) of 185°C or higher, and more preferably 190°C or higher, as measured with use of a differential thermogravimetric analyzer under conditions of a sample weight of 10 mg, a temperature rise condition of 10°C/min, and a nitrogen flow (200 mL/min), from the viewpoint of volatility.

The inkjet curable ink composition preferably has a viscosity of 5 mPa·s or more and 150 mPa·s or less, more preferably 10 mPa·s or more and 80 mPa·s or less, and even more preferably 10 mPa·s or more and 40 mPa·s or less, as measured at 25°C with use of a dynamic viscoelasticity measuring device ("Rheosol-G5000" manufactured by UBM). Note that the inkjet curable ink composition may be applied by the inkjet method by being heated to reduce the viscosity.

Further, the inkjet curable ink composition preferably has a surface tension of 15 mN/m or more and 35 mN/m or less, and more preferably 20 mN/m or more and 30 mN/m or less, as measured with a dynamic wettability tester at 25°C.

Suitable examples of the inkjet curable ink composition include an optical element-sealing curable composition used as a sealant for an optical element such as an organic electroluminescence (hereinafter referred to as organic EL) element or an organic thin-film solar cell element, an optical member adhesive used for, for example, bonding optical lenses, a stereolithography curable composition used in manufacturing various components and members by a 3D printer, and a nanoimprint curable composition used in forming an ultrafine circuit pattern of a semiconductor.

Examples of a method for using the curable composition according to an aspect of the present invention as the optical element-sealing curable composition to manufacture an optical element include a method including a step of applying the optical element-sealing curable composition to at least one of two substrates by an inkjet method, a step of curing the applied optical element-sealing curable composition by light irradiation and/or heating, and a step of bonding the two substrates.

In the step of applying the optical element-sealing curable composition to at least one of the two substrates, the optical element-sealing curable composition may be applied to the entire surface of the substrate(s) or may be applied to a part of the substrate(s). For example, in a case where an organic EL element is manufactured as an optical element, the shape of a sealant part formed by application of the optical element-sealing curable composition is not particularly limited as long as the shape can protect, from outside air, a laminate having an organic light-emitting material layer sandwiched between two substrates. That is, the shape may be a shape that completely covers the laminate, or may be a pattern shape that closes a peripheral part of the laminate.

The step of curing the optical element-sealing curable composition by light irradiation and/or heating may be carried out before the step of bonding the two substrates, or may be carried out after the step of bonding the two substrates. In a case where the step of curing the optical element-sealing curable composition by light irradiation and/or heating is carried out before the step of bonding the two substrates, the two substrates may be bonded within a pot life (for example, 1 minute) until the substrates can no longer be bonded due to proceeding of a curing reaction after light irradiation and/or heating.

Further, the step of curing the applied optical element-sealing curable composition by light irradiation and/or heating may be carried out by the foregoing ultraviolet irradiation means and/or heating means.

The curable composition according to an aspect of the present invention is not particularly limited in use, is applicable to products (components/members) in various fields which products may be made of resin, and can be used as a raw material for materials in the electric/electronic field, the optical field, the architectural field, the civil engineering field, the automobile and aircraft fields, and the medical field, and other materials for daily necessities/miscellaneous goods.

For example, examples of components/members and materials in the electric/electronic field include a resincoated copper foil, a prepreg, a copper-clad laminate, a printed wiring board, a solder resist ink, an anisotropic conductive film, an anisotropic conductive paste, an interlayer insulating material, an adhesive, a pressure-sensitive adhesive, a seal material, a sealant (sealing material), a sealing sheet, an insulating material, a thermally conductive material, a hot melt material, a paint, and a potting agent, and more specific examples thereof include: sealing materials (sealants) for printed wiring boards and electronic components, such as interlayer insulating films and wiring coating films, sealing materials (sealants) for image display devices, sealing sheets for image display devices, sealing materials (sealants) for organic EL display elements, sealing sheets for organic EL display elements, and layerforming materials; materials for forming display devices, such as color filters, polarizing plates, display materials, resist materials, and alignment films; materials for forming semiconductor devices, such as resist materials and buffer coat films; materials for forming optical components, such as lenses, holograms, optical waveguides, optical circuits, optical circuit components, and antireflection films; and coating agents such as surface protective films of semiconductor elements and organic thin-film elements (e.g., organic electroluminescence elements and organic thin-film solar cell elements), hard coat agents, antifouling films, and antireflection films.

Further examples of the components/members and materials in the electric/electronic field include materials for organic electronics elements such as organic EL elements, organic transistors, and solar cells, materials for forming rigid wiring boards and flexible printed wiring boards for semiconductor mounting, mounting materials for semiconductor mounting, adhesives for flexible printed wiring boards, sealants for semiconductors, sealants for solar cells, insulating films for semiconductors, coverlay films for flexible printed circuit protection, and coating agents for wiring coating.

Examples of the materials in the optical field include lenses, prisms, films, core materials for optical fibers, clad materials, abrasion-resistant coating agents for plastic lenses, materials for stereolithography, optical waveguides, filters, image display materials, lens arrays, sealants and reflector materials for optical semiconductor elements, light guide plates, light diffusion plates, diffraction elements, and optical adhesives. For example, lenses and prisms are not particularly limited as long as the lenses and the prisms use surface refraction.

Further, the materials in the optical field can be used as: materials for lenses of camera modules, LiDAR modules, and still cameras; adhesives for finder prisms, target prisms, finder covers, light-receiving sensor parts, photographing lenses, projection lenses of projection televisions, etc.; optical fiber materials surrounding optical switches and optical connectors in optical communication systems; and sealants and adhesives surrounding optical passive components, optical circuit components, optoelectronic integrated circuits, etc. Examples of a bonding location of a camera module include a space between an image sensor (imaging element) such as CMOS or CCD and a substrate, a space between a cut filter and a substrate, a space between a substrate and a housing, a space between a housing and a cut filter, and a space between a housing and a lens unit.

Examples of the lenses and the prisms include: spherical lenses, the surfaces of which are composed of spherical surfaces, such as convex lenses like biconvex lenses, planoconvex lenses, and convex meniscus lenses, and concave lenses like biconcave lenses, plano-concave lenses, and concave meniscus lenses; aspherical lenses such as lenses having aspherical surfaces like symmetric paraboloids, ellipsoids, hyperboloids, and multidimensional curved surfaces (e.g., quartic curved surfaces), and lenses having free-form curved surfaces without axes of symmetry; cylindrical lenses that are semi-cylindrical, such as cylindrical surface lenses; toroidal lenses having toroidal surfaces with different radii of curvature in the vertical and horizontal directions like the surface of a donut; thin Fresnel lenses in which annular prisms having a reflecting surface that is inclined in the same manner as inclination of a curve of a thick convex lens and that changes the traveling direction of light and a refracting surface that transmits light are arranged concentrically on a plane, and these prisms become continuously smaller or larger toward the center; diffractive lenses in which a fine relief having a depth of approximately the wavelength of light is formed concentrically; and prisms having two or more optical planes, at least one pair of which is substantially non-parallel.

Examples of the films include reflective films that reflect light in an undesired wavelength range while transmitting light in a desired wavelength range, by treating an inorganic compound such as MgF₂ or SiO₂ by a vacuum deposition method, a sputtering method, or a CVD method, and/or allowing a light absorber to coexist.

Examples of the materials in the architectural field include: joint seal materials, coating materials, and primers for exterior materials such as various metal panels and siding boards; seal materials, adhesives, injection materials, damping materials, soundproofing materials, electromagnetic wave shielding conductive materials, and putty materials for use between exterior materials/base materials/ceiling materials and interior materials; adhesives for bonding tiles and stones to exterior wall materials and base materials; adhesives and pressure-sensitive adhesives for bonding wood flooring materials, polymer material-based floor sheets, and floor tiles to various floors; and injection materials for repairing cracks in various exterior materials and interior materials.

Examples of the materials in the civil engineering field include joint seal materials, coating materials, primers, paints, putty materials, injection materials, spraying materials, and molding materials for various concrete products such as roads, bridges, tunnels, and breakwaters.

Examples of the materials in the automobile and aircraft fields include: structural materials, fiber-reinforced composite materials, adhesives for bodies and components, seal materials, coating materials, cushioning materials, damping materials, soundproofing materials, and spraying materials; adhesives, pressure-sensitive adhesives, coating materials, and foam materials for automobile interiors; and seal materials, adhesives, and coating materials for steel plate joints.

Further, examples of uses of a substrate, the surface of which is provided with a coating layer formed from a cured product of the curable composition according to an aspect of the present invention, include members of various pieces of industrial equipment such as transport aircrafts (automobiles, aircrafts, etc.) and electronic/electric devices (e.g., sliding members such as cylinders, pistons, and bearings).

Examples of the materials in the medical field include artificial bones, dental impression materials, medical rubber materials, medical pressure-sensitive adhesives, and medical device seal materials.

Examples of the other materials include paints applied to substrates such as metals, resin films, glass, paper, and wood.

Aspects of the present invention can also be expressed as follows:
As described above, the inventors of the present invention found that a desired object can be achieved by adopting a curable composition containing the epoxy-oxetane compound represented by Chemical Formula (I), and arrived at the present invention.

That is, the first invention is a curable composition containing an epoxy-oxetane compound represented by Chemical Formula (I). 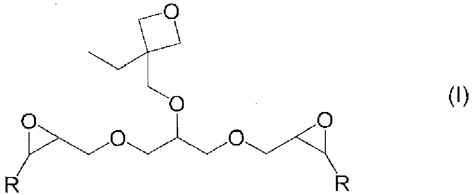 wherein Rs are identical or different and each represent a hydrogen atom or an alkyl group having 1 to 3 carbon atoms.

The curable composition may further contain a cationic polymerization initiator.

The second invention is a cured product obtained by curing the curable composition of the first invention.

The third invention is an adhesive containing the curable composition of the first invention.

The fourth invention is a sealant containing the curable composition of the first invention.

### Examples

The following description will discuss the present invention in more detail with use of Examples and Comparative Examples, but the present invention is not limited thereto.

Main raw materials used are as follows.
- Allyl glycidyl ether (manufactured by Tokyo Chemical Industry Co., Ltd.)
- Allyl alcohol (manufactured by Fujifilm Wako Pure Chemical Corporation)
- Potassium tert-butoxide (manufactured by Tokyo Chemical Industry Co., Ltd.)
- 3-ethyl-3-methanesulfonyloxymethyloxetane (synthesized in accordance with the method disclosed in Japanese Patent Application Publication Tokukai No. 2007-332294)
- Sodium hydroxide (manufactured by Fujifilm Wako Pure Chemical Corporation)
- *m*-chloroperoxybenzoic acid (manufactured by Tokyo Chemical Industry Co., Ltd.)
- 1,2,3-glycidyloxypropane (manufactured by Anhui Xinyuan Technology Co., Ltd., trade name "XY633", compound represented by Chemical Formula (IV))
- Glycerin (manufactured by Fujifilm Wako Pure Chemical Corporation)
- 3-ethyl-3-hydroxymethyloxetane (manufactured by Ube Industries, Ltd.)
- Allyl chloride (manufactured by Fujifilm Wako Pure Chemical Corporation)
- 2-chloromethyloxirane (manufactured by Osaka Soda Co., Ltd.)
- Allyl chloride (manufactured by Tokyo Chemical Industry Co., Ltd.)
- Sodium iodide (manufactured by Fujifilm Wako Pure Chemical Corporation)
- Potassium iodide (manufactured by Fujifilm Wako Pure Chemical Corporation) 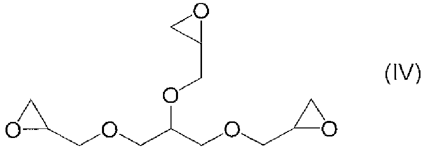 (C) Thermal cationic polymerization initiator
- Dibenzylmethyl-p-hydroxyphenylsulfonium hexafluoroantimonate (manufactured by Sanshin Chemical Industry Co., Ltd., "San-Aid SI-100L (trade name)")

Methods for measuring viscosity, an elastic modulus, a loss tangent, a flexural modulus, and flexural strength, which methods are evaluation tests adopted in Examples and Comparative Examples, are as follows.

### [Measurement of viscosity]

For epoxy-oxetane compounds (including epoxy compounds and oxetane compounds) used in Examples and Comparative Examples (described later), the viscosity at 25°C was measured with use of a dynamic viscoelasticity measuring device ("Rheosol-G5000" manufactured by UBM).

It is determined that an epoxy-oxetane compound having a smaller viscosity value (lower viscosity) has more excellent workability during preparation of a curable composition and during use of the curable composition as an adhesive.

### [Measurement of elastic modulus and loss tangent]

Curable compositions prepared in Examples and Comparative Examples (described later) were cured at 65°C for 2 hours and further heated and cured at 150°C for 2 hours. For the obtained cured product (test piece: 30 mm in length × 10 mm in width × 1 mm in thickness), a storage elastic modulus G' (MPa) and a loss tangent tan δ at 25°C were measured with use of the dynamic viscoelasticity measuring device ("Rheosol-G5000" manufactured by UBM) (frequency: 1 Hz).

It is determined that the cured product having a larger storage elastic modulus G' value (higher elasticity) has more excellent rigidity, and the cured product having a smaller loss tangent tan δ value forms a more uniform three-dimensional network with fewer structural defects in the cured product.

### [Measurement of flexural modulus and flexural strength]

The curable compositions prepared in Examples and Comparative Examples (described later) were cured at 65°C for 2 hours and further heated and cured at 150°C for 2 hours. For the obtained cured product (test piece: 80 mm in length × 25 mm in width × 1 mm in thickness), a flexural modulus (GPa) and flexural strength (MPa) were measured in accordance with JIS K7203.

It is determined that the cured product having a larger flexural modulus (higher elasticity) has more excellent rigidity, and the cured product having higher flexural strength has more excellent mechanical strength.

### <Synthesis of epoxy-oxetane compound represented by Chemical Formula (I-1)>

### [Synthesis Example 1]

A 3-neck flask having a capacity of 5 L was charged with 2,178 g (37,500 mmol) of allyl alcohol and 308.58 g (2,750 mmol) of potassium tert-butoxide. After heating to 40°C, 285.35 g (2,500 mmol) of allyl glycidyl ether was added dropwise, and the mixture thus obtained was stirred at 60°C for 14 hours. Thereafter, a reaction liquid was cooled to 25°C or lower, then the reaction liquid was filtered to remove a solid, and a filtrate was distilled off under reduced pressure. The obtained concentrated liquid was purified by distillation to obtain, as a colorless transparent liquid, 391.19 g of a bifunctional olefin compound represented by Chemical Formula (II-1) and having a hydroxy group (yield: 91%).

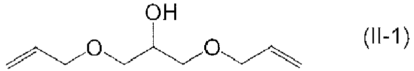

### [Synthesis Example 2]

A 4-neck flask having a capacity of 5 L was charged with 344.44 g (2,000 mmol) of the colorless transparent liquid obtained in Synthesis Example 1, 1,500 g of dimethyl sulfoxide, and 114.96 g (2,874 mmol) of sodium hydroxide. After heating to 60°C, 427.35 g (2,200 mmol) of 3-ethyl-3-methanesulfonyloxymethyloxetane was added dropwise, and the mixture thus obtained was stirred at 70°C for 8 hours. Thereafter, a reaction liquid was cooled to 25°C or lower, a solid was filtered off, and a filtrate was extracted with 2,000 mL of ethyl acetate. Subsequently, after washing with 2,000 mL of water, an organic layer was concentrated under reduced pressure. The obtained concentrated liquid was purified by distillation to obtain, as a colorless transparent liquid, 336.92 g of an olefin-oxetane compound represented by Chemical Formula (Ia-1) (yield: 62%).

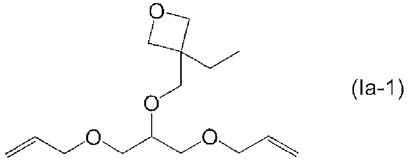

### [Synthesis Example 3]

A 3-neck flask having a capacity of 1 L was charged with 54.07 g (200.0 mmol) of the colorless transparent liquid obtained in Synthesis Example 2, 86.29 g (500.0 mmol) of *m-*chloroperoxybenzoic acid, and 500 g of chloroform, and the mixture thus obtained was stirred at 30°C for 12 hours. This reaction liquid was filtered to remove a solid, washing with 500 mL of a 10 wt% aqueous potassium carbonate solution and 500 mL of water was carried out, and then an organic layer was concentrated under reduced pressure. The obtained concentrate was purified by silica gel column chromatography (ethyl acetate/hexane = 1/3 (volume ratio)) to obtain 26.49 g of a pale yellow transparent liquid (yield: 44%).

¹H-NMR spectral data of this pale yellow transparent liquid were as follows.
- ¹H-NMR (CDCl₃) δ: 4.46 (d, 2H), 4.37 (d, 2H), 3.79 (m, 2H), 3.74 (t, 2H), 3.61 (m, 5H), 3.41 (m, 2H), 3.13 (quin., 2H), 2.79 (t, 2H), 2.60 (m, 2H), 1.74 (q, 2H), 0.88 (t, 3H).

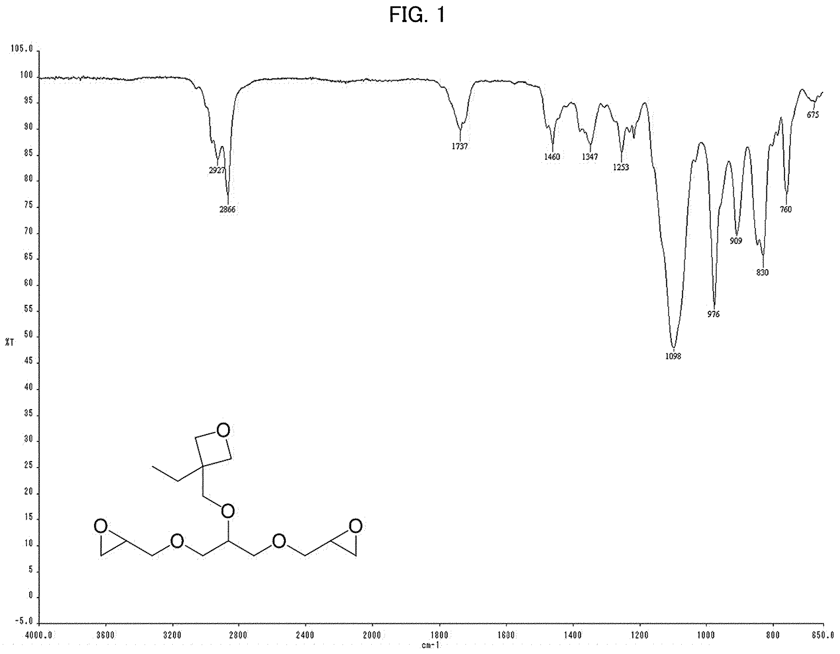

IR spectral data of the pale yellow transparent liquid were as shown in the chart illustrated in Fig. 1.

From these spectral data, the obtained pale yellow transparent liquid was identified as the epoxy-oxetane compound represented by Chemical Formula (I-1).

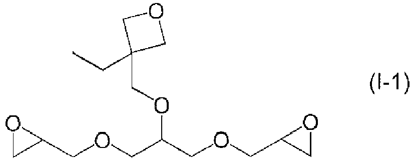

### <Synthesis of oxetane compound represented by Chemical Formula (V)>

### [Synthesis Example 4]

A 3-neck flask having a capacity of 500 mL was charged with 18.42 g (200.0 mmol) of glycerin, 180.0 g of dimethyl sulfoxide, and 22.20 g (555.0 mmol) of sodium hydroxide. After heating to 60°C, 104.90 g (540.0 mmol) of 3-ethyl-3-methanesulfonyloxymethyloxetane was added dropwise, and the mixture thus obtained was stirred at 70°C for 8 hours. Thereafter, a reaction liquid was cooled to 25°C or lower, a solid was filtered off, and a filtrate was extracted with 200 mL of ethyl acetate. Subsequently, after washing with 60 mL of water, an organic layer was concentrated under reduced pressure.

The obtained concentrate was purified by silica gel column chromatography (ethyl acetate/hexane = 2/3 (volume ratio)) to obtain 8.50 g of a colorless transparent liquid (yield: 11%).

¹H-NMR spectral data of this colorless transparent liquid were as follows.
- ¹H-NMR (CDCl₃) δ: 4.44 (d, 6H), 4.35 (d, 6H), 3.72 (s, 2H), 3.66 (quin., 1H), 3.57 (m, 6H), 3.55 (d, 2H), 1.74 (q, 6H), 0.88 (t, 9H).

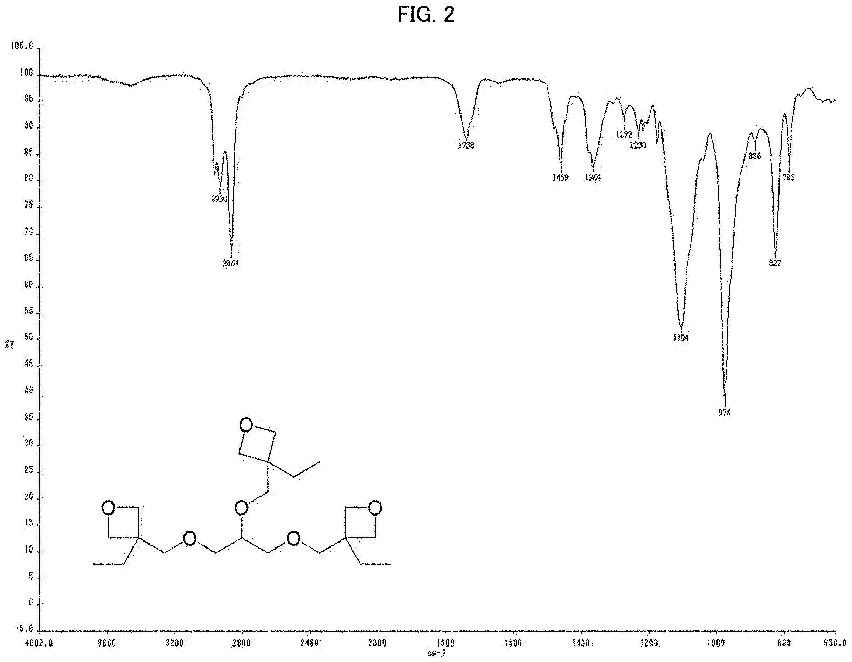

IR spectral data of the colorless transparent liquid were as shown in the chart illustrated in Fig. 2.

From these spectral data, the obtained colorless transparent liquid was identified as an oxetane compound represented by Chemical Formula (V).

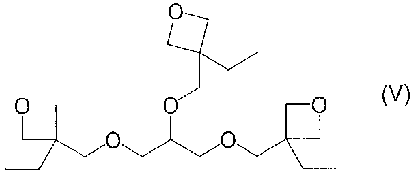

### <Synthesis of epoxy-oxetane compound represented by Chemical Formula (VIII)>

### [Synthesis Example 5]

A 3-neck flask having a capacity of 1 L was charged with 174.24 g (1,500 mmol) of 3-ethyl-3-hydroxymethyloxetane and 8.00 g (200.0 mmol) of sodium hydroxide. After heating to 45°C, 114.14 g (1,000 mmol) of allyl glycidyl ether was added dropwise, and the mixture thus obtained was stirred at 50°C for 12 hours. Thereafter, after a reaction liquid was cooled to 25°C or lower, the cooled reaction liquid was extracted with 750 mL of chloroform, washing with 500 mL of water was carried out, and then an organic layer was concentrated under reduced pressure to obtain, as a pale yellow transparent liquid, 223.4 g of a compound represented by Chemical Formula (VI) (yield: 97%).

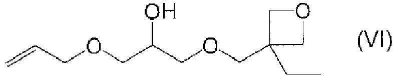

### [Synthesis Example 6]

A 3-neck flask having a capacity of 2 L was charged with 184.73 g (750.0 mmol) of the pale yellow transparent liquid obtained in Synthesis Example 5, 750.00 g of dimethyl sulfoxide, 45.00 g (1,125.0 mmol) of sodium hydroxide, and 5.62 g (37.5 mmol) of sodium iodide. After heating to 50°C, 86.10 g (1,125.0 mmol) of allyl chloride was added dropwise, and the mixture thus obtained was stirred at 50°C for 8 hours. Thereafter, a reaction liquid was cooled to 25°C or lower, a solid was filtered off, and a filtrate was extracted with 1,500 mL of ethyl acetate. Subsequently, after washing with 1,500 mL of water, an organic layer was concentrated under reduced pressure. The obtained concentrated liquid was purified by distillation to obtain, as a colorless transparent liquid, 121.67 g of a compound represented by Chemical Formula (VII) (yield: 60%).

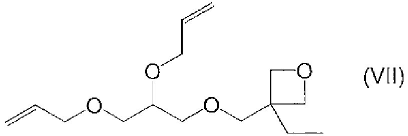

### [Synthesis Example 7]

A 3-neck flask having a capacity of 500 mL was charged with 27.03 g (100.0 mmol) of the colorless transparent liquid obtained in Synthesis Example 6, 43.14 g (250.0 mmol) of *m-*chloroperoxybenzoic acid, and 250 g of chloroform, and the mixture thus obtained was stirred at 30°C for 12 hours. This reaction liquid was filtered to remove a solid, washing with 250 mL of a 10 wt% aqueous potassium carbonate solution and 250 mL of water was carried out, and then an organic layer was concentrated under reduced pressure. The obtained concentrate was purified by silica gel column chromatography (ethyl acetate/hexane = 1/3 (volume ratio)) to obtain 12.02 g of a pale yellow transparent liquid (yield: 40%).

¹H-NMR spectral data of this pale yellow transparent liquid were as follows.
- ¹H-NMR (CDCl₃) δ: 4.46 (d, 2H), 4.36 (d, 2H), 3.91 (m, 1H), 3.76 (m, 2H), 3.60 (m, 7H), 3.41 (m, 1H), 3.15 (m, 2H), 2.78 (m, 2H), 2.61 (m, 2H), 1.74 (q, 2H), 0.89 (t, 3H).

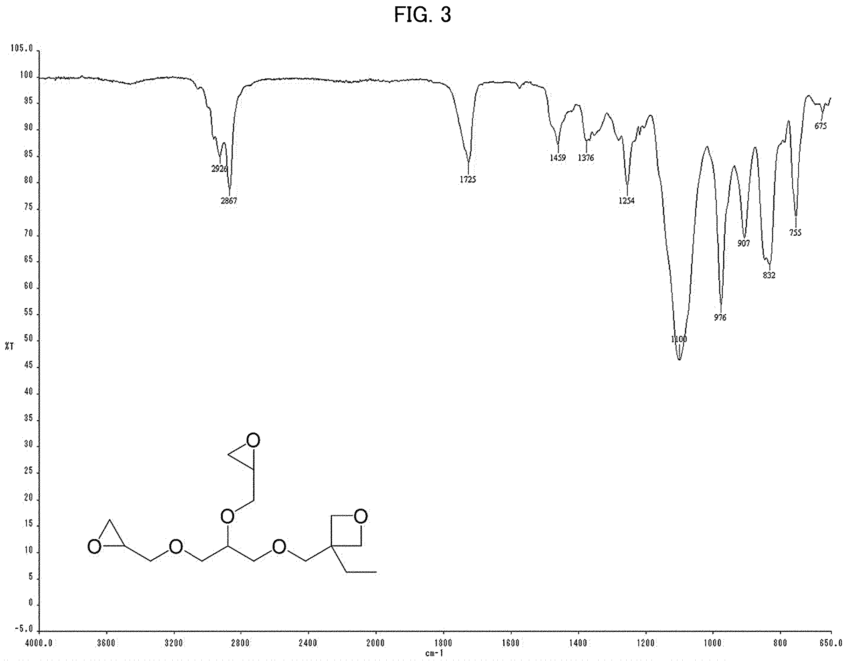

IR spectral data of the pale yellow transparent liquid were as shown in the chart illustrated in Fig. 3.

From these spectral data, the obtained pale yellow transparent liquid was identified as an epoxy-oxetane compound represented by Chemical Formula (VIII).

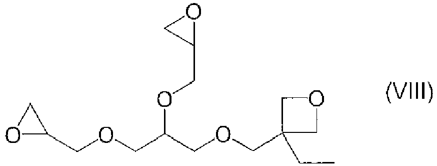

### <Synthesis of epoxy-oxetane compound represented by Chemical Formula (XI)>

### [Synthesis Example 8]

A 3-neck flask having a capacity of 1 L was charged with 278.78 g (2,400.0 mmol) of 3-ethyl-3-hydroxymethyloxetane and 38.40 g (960.0 mmol) of sodium hydroxide. After heating to 47°C, 74.02 g (800.0 mmol) of 2-chloromethyloxirane was added dropwise, and the mixture thus obtained was stirred at 60°C for 4 hours. Thereafter, after a reaction liquid was cooled to 25°C or lower, the cooled reaction liquid was extracted with 900 mL of chloroform, washing with 500 mL of water was carried out, and then an organic layer was concentrated under reduced pressure. A concentrated liquid was purified by distillation to obtain, as a colorless transparent liquid, 87.51 g of a compound represented by Chemical Formula (IX) (yield: 38%).

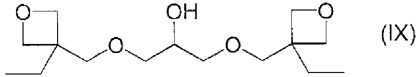

### [Synthesis Example 9]

A 3-neck flask having a capacity of 300 mL was charged with 40.37 g (140.0 mmol) of the colorless transparent liquid obtained in Synthesis Example 8, 8.12 g (203.0 mmol) of sodium hydroxide, and 1.16 g (7.0 mmol) of potassium iodide. After heating to 67°C, 13.93 g (182.0 mmol) of allyl chloride was added dropwise, and the mixture thus obtained was stirred at 70°C for 2 hours. Thereafter, a reaction liquid was cooled to 25°C or lower, a solid was filtered off, and a filtrate was extracted with 80 mL of ethyl acetate. Subsequently, after washing with 40 mL of water, an organic layer was concentrated under reduced pressure to obtain, as a colorless transparent liquid, 44.79 g of a compound represented by Chemical Formula (X) (yield: 97%).

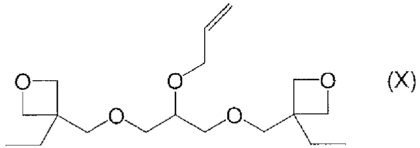

### [Synthesis Example 10]

A 3-neck flask having a capacity of 500 mL was charged with 42.70 g (130.0 mmol) of the colorless transparent liquid obtained in Synthesis Example 9, 22.43 g (130.0 mmol) of *m-*chloroperoxybenzoic acid, and 300 g of chloroform, and the mixture thus obtained was stirred at 30°C for 12 hours. Subsequently, this reaction liquid was filtered to remove a solid, and washing with 150 mL of a 10 wt% aqueous potassium carbonate solution and 150 mL of water was carried out. An organic layer was concentrated under reduced pressure to obtain 38.44 g of a colorless transparent liquid (yield: 86%).

¹H-NMR spectral data of this colorless transparent liquid were as follows.
- ¹H-NMR (CDCl₃) δ: 4.44 (d, 4H), 4.35 (d, 4H), 3.89 (dd, 1H), 3.72 (quin., 1H), 3.56 (m, 9H), 3.12 (quin, 1H), 2.77 (t, 1H), 2.60 (dd, 1H), 1.72 (q, 4H), 0.87 (t, 6H).

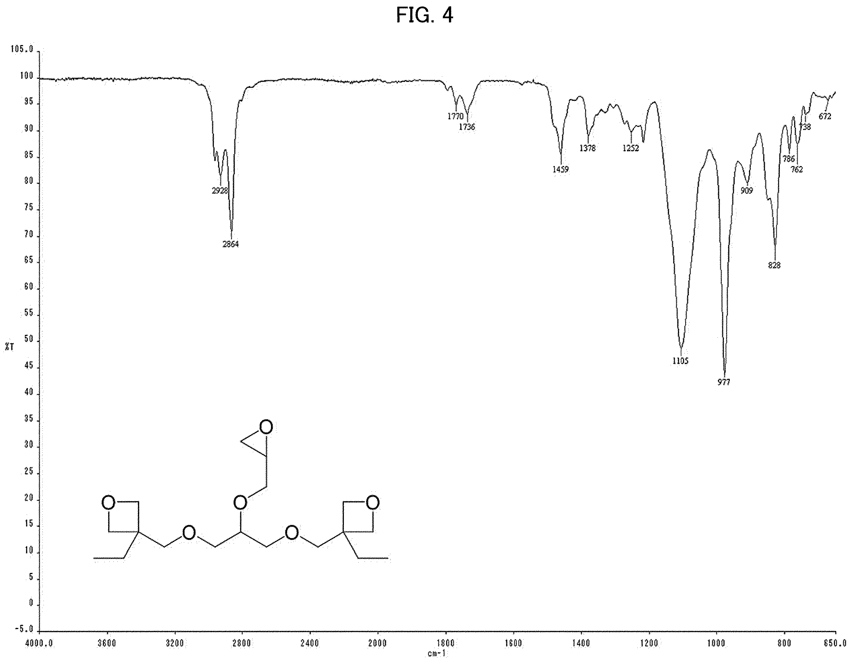

IR spectral data of the colorless transparent liquid were as shown in the chart illustrated in Fig. 4.

From these spectral data, the obtained colorless transparent liquid was identified as an epoxy-oxetane compound represented by Chemical Formula (XI).

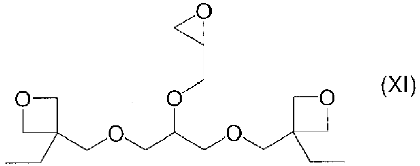

### [Synthesis Example 11]

A 3-neck eggplant flask having a capacity of 1 L was charged with 49.26 g (200.0 mmol) of the pale yellow transparent liquid obtained in Synthesis Example 5, 200.00 g of dimethyl sulfoxide, 10.40 g (260.0 mmol) of sodium hydroxide, and 1.50 g (10.0 mmol) of sodium iodide. After heating to 50°C, 50.51 g (260.0 mmol) of 3-ethyl-3-methanesulfonyloxymethyloxetane was added dropwise, and the mixture thus obtained was stirred at 50°C for 8 hours. Thereafter, a reaction liquid was cooled to 25°C or lower, a solid was filtered off, and a filtrate was extracted with 400 mL of ethyl acetate. Subsequently, after washing with 400 mL of water, an organic layer was concentrated under reduced pressure. The obtained concentrated liquid was purified by distillation to obtain, as a colorless transparent liquid, 26.32 g of a compound represented by Chemical Formula (XII) (yield: 40%).

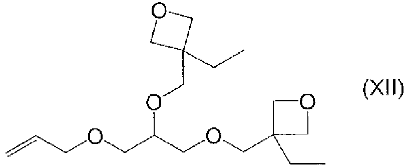

### [Synthesis Example 12]

A 3-neck eggplant flask having a capacity of 1 L was charged with 26.28 g (80.0 mmol) of the colorless transparent liquid obtained in Synthesis Example 11, 16.57 g (96.0 mmol) of m-chloroperoxybenzoic acid, and 500 g of chloroform, and the mixture thus obtained was stirred at 30°C for 12 hours. This reaction liquid was filtered to remove a solid, washing with 100 mL of a 10 wt% aqueous potassium carbonate solution and 100 mL of water was carried out, and then an organic layer was concentrated under reduced pressure. The obtained concentrate was purified by silica gel column chromatography (ethyl acetate/hexane = 2/3 (volume ratio)) to obtain 17.05 g of a pale yellow transparent liquid (yield: 62%).

¹H-NMR spectral data of this pale yellow transparent liquid were as follows.
- ¹H-NMR (CDCl₃) δ: 4.43 (d, 4H), 4.32 (d, 4H), 3.77 (dd, 1H), 3.72 (d, 2H), 3.56 (m, 7H), 3.39 (m, 1H), 3.12 (quin., 1H), 2.78 (t, 1H), 2.59 (dd, 1H), 1.72 (q, 4H), 0.87 (t, 6H).

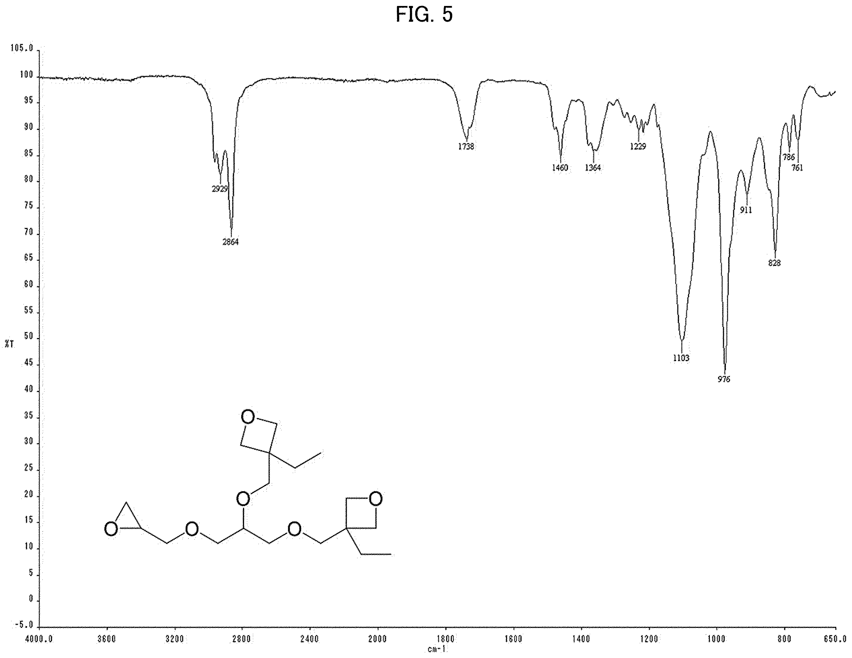

IR spectral data of the pale yellow transparent liquid were as shown in the chart illustrated in Fig. 5.

From these spectral data, the obtained pale yellow transparent liquid was identified as an epoxy-oxetane compound represented by Chemical Formula (XIII).

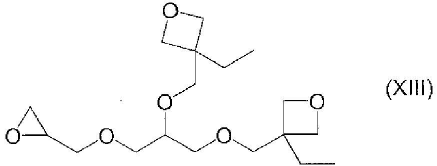

### <Evaluation of cured product>

### [Example 1]

100 parts by weight of the epoxy-oxetane compound synthesized in Synthesis Example 3 and represented by Chemical Formula (I-1) and 0.2 parts by weight of a thermal cationic polymerization initiator were uniformly mixed to prepare a curable composition.

The foregoing evaluation tests were carried out with respect to this curable composition and a cured product obtained by curing the curable composition. The obtained test results were as shown in Table 1.

### [Comparative Examples 1 to 5]

Curable compositions having the compositions shown in Table 1 were prepared in the same manner as in Example 1, and the foregoing evaluation tests were carried out with respect to those curable compositions and cured products obtained by curing the curable compositions. The obtained test results were as shown in Table 1.

| | | Example 1 | Comparative Example 1 | Comparative Example 2 |
|---|---|---|---|---|
| Formulation composition (part by weight) | Epoxy-oxetane compound represented by Chemical Formula (I-1) | 100 | | |
| | Epoxy compound represented by Chemical Formula (IV) | | 100 | |
| | Oxetane compound represented by Chemical Formula (V) | | | 100 |
| | Epoxy-oxetane compound represented by Chemical Formula (VIII) | | | |
| | Epoxy-oxetane compound represented by Chemical Formula (XI) | | | |
| | Epoxy-oxetane compound represented by Chemical Formula (XIII) | | | |
| | San-Aid SI-100L | 0.2 | 0.2 | 0.2 |
| Evaluation test | Viscosity at 25°C (mPa·s) | 48 | 225 | 211 |
| | Storage elastic modulus G' at 25°C (MPa) | 1.06 | Unevaluable due to failure to obtain cured product | Unevaluable due to failure to obtain cured product |
| | Loss tangent tan δ | 0.060 | | |
| | Flexural modulus (GPa) | 2.08 | | |
| | Flexural strength (MPa) | 44.4 | | |

| | | Comparative Example 3 | Comparative Example 4 | Comparative Example 5 |
|---|---|---|---|---|
| Formulation composition (part by weight) | Epoxy-oxetane compound represented by Chemical Formula (I-1) | | | |
| | Epoxy compound represented by Chemical Formula (IV) | | | |
| | Oxetane compound represented by Chemical Formula (V) | | | |
| | Epoxy-oxetane compound represented by Chemical Formula (VIII) | 100 | | |
| | Epoxy-oxetane compound represented by Chemical Formula (XI) | | 100 | |
| | Epoxy-oxetane compound represented by Chemical Formula (XIII) | | | 100 |
| | San-Aid SI-100L | 0.2 | 0.2 | 0.2 |
| Evaluation test | Viscosity at 25°C (mPa·s) | 96 | 95 | 102 |
| | Storage elastic modulus G' at 25°C (MPa) | 0.87 | 1.01 | 0.71 |
| | Loss tangent tan δ | 0.070 | 0.837 | 0.059 |
| | Flexural modulus (GPa) | 1.73 | 1.50 | 1.43 |
| | Flexural strength (MPa) | 37.9 | 35.6 | 30.5 |

Table 1 confirms that the epoxy-oxetane compound contained in the curable composition according to an aspect of the present invention has extremely low viscosity, and thus has excellent workability during preparation of the epoxy-oxetane compound and during use of the epoxy-oxetane compound as a curable adhesive. It is also confirmed that the curable composition according to an aspect of the present invention yields a cured product by curing due to exhibition of a crosslinking function, and achieves excellent mechanical strength of the cured product because the values of the storage elastic modulus G', the flexural modulus, and the flexural strength of the cured product are large despite low viscosity of the curable composition. A cured product obtained by curing the curable composition according to an aspect of the present invention has a small loss tangent tan δ value, and thus is estimated to form a uniform three-dimensional network with few structural defects in the cured product.

### Industrial Applicability

As compared with a conventional curable composition, the curable composition of the present invention makes it possible to provide a curable composition having curability, low viscosity, and mechanical strength, and a cured product of the curable composition, and the present invention is highly industrially applicable.

## Claims

1. A curable composition comprising an epoxy-oxetane compound represented by Chemical Formula (I): 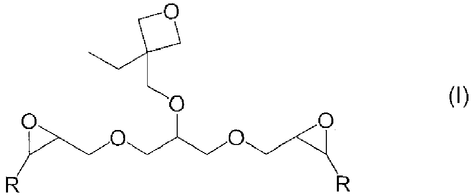 wherein Rs are identical or different and each represent a hydrogen atom or an alkyl group having 1 to 3 carbon atoms.

2. The curable composition according to claim 1, further comprising a cationic polymerization initiator.

3. A cured product obtained by curing the curable composition according to claim 1 or 2.

4. An adhesive comprising the curable composition according to claim 1 or 2.

5. A sealant comprising the curable composition according to claim 1 or 2.
